# EUROPEAN PATENT APPLICATION

(11) **EP 3 299 363 A1**
(43) Date of publication of application: **28.03.2018**
(21) Application number: 17190743.9
(22) Date of filing: 13.09.2017
(51) Int. Cl.: C07D 251/04

(54) **1, 3, 5 - TRIAZINAN COMPOUNDS**

(30) Priority: 23.09.2016 US 201662399135 P
(71) Applicant: BASF SE, 67056 Ludwigshafen am Rhein (DE)
(72) Inventor: Panchenko, Alexander, 67071 Ludwigshafen (DE); Kozicki, Artur, 67098 Bad Duerkheim (DE); Busch, Ralph, 67549 Worms (DE); Gutfrucht, Norbert, 67466 Lambrecht (DE); Zarbakhsh, Sirus, 67117 Limburgerhof (DE); Sundar, Rajendra Andrew, Morristown, NJ New Jersey 07960 (US)
(74) Representative: BASF IP Association

(57) **Abstract**

A 1, 3, 5 - triazinan compound of formula I 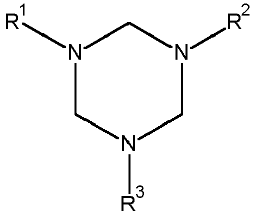 wherein the groups R¹ to R³ are organic groups and wherein at least one of the groups R¹ to R³ is a hydroxyl functionalized group comprising at least one hydroxyl group and wherein at least one of the groups R¹ to R³ is a tertiary amino functionalized group comprising at least one tertiary amino group.

## Description

The invention relates to a 1, 3, 5 - triazinan compound of formula I 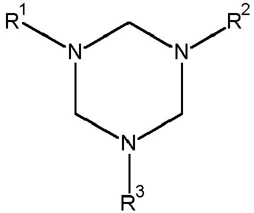 wherein the groups R¹ to R³ are organic groups and wherein at least one of the groups R¹ to R³ is a hydroxyl functionalized group comprising at least one hydroxyl group and wherein at least one of the groups R¹ to R³ is a tertiary amino functionalized group comprising at least one tertiary amino group.

Compounds with tertiary amino groups are suitable as catalysts for the preparation of polyurethanes and polyureas by reacting polyisocyanates and polyols, respectively polyamines. One well know catalyst is N, N-dimethylcyclohexylamine. Catalysts like N, N-dimethylcyclohexylamine are volatile and leak from the reacting composition at high temperatures and thus cause undesired side reactions in the gas phase, for example decomposition of blowing agents in case of foamed polyurethanes. Furthermore such catalysts might later leak from the polyurethane prepared, for example a polyurethane foam and thus cause fogging, contamination of the environment and/or unpleasant odor.

The prior art discloses catalysts which are made to overcome this problem. In US 5,688,834 a polyamine is disclosed which is less volatile than N, N-dimethylcyclohexylamine due to its higher molecular weight. From US 4,113,945 catalysts with a triazinan ring system and three substituents with tertiary amino groups are known. Such catalysts may have high catalytic activity due to the high number of tertiary amino groups, but still have volatility.

Alternatively, the prior art suggests catalysts that react with polyisocyanates, polyols or polyamines and thus become part of the polymer itself. EP-A 1262500 teaches reactive catalysts with a primary amino or a hydroxyl group as reactive group and a cyclic or bicyclic system with a catalytically active tertiary amino group. Similar structures are disclosed in EP-A 2657237.

Reactive catalysts may overcome the above problems caused by volatility if all or nearly all of the catalyst is bonded to the polymer. However, reactive catalysts should nevertheless have high catalytic activity. Hence reactive catalysts are desired that are completely bonded to the polymer and still have high reactivity. In addition, synthesis of reactive catalysts should be as easy as possible. In particular, the process of synthesis should give such reactive catalysts in high yield and high selectivity at low costs.

It was an object of the present invention to find reactive catalysts that fulfill the above requirements as good as possible.

Accordingly, the above 1, 3, 5 - triazinan compound of formula I has been found. In addition, a process for the synthesis of such 1, 3, 5 - triazinan compound and the use of the above 1, 3, 5 - triazinan as catalyst for the preparation of polyurethanes or polyureas have been found.

To the 1, 3, 5 - triazinan compound of formula I

R¹ to R³ are organic groups wherein at least one of the groups R¹ to R³ is a hydroxyl functionalized group comprising at least one hydroxyl group and wherein at least one of the groups R¹ to R³ is a tertiary amino functionalized group comprising at least one tertiary amino group.

In a preferred embodiment the hydroxyl functionalized groups comprise one hydroxyl group but no tertiary amino group and the tertiary amino functionalized groups comprise one tertiary amino group but no hydroxyl group.

Preferably, R¹ to R³ are non aromatic hydrocarbon groups of at maximum 20 carbon atoms, in particular of at maximum 10 carbon atoms, which may comprise ether groups and wherein at least one of the groups R¹ to R³ is a hydroxyl functionalized group comprising one hydroxyl group but no tertiary amino group and at least one of the groups R¹ to R³ is a tertiary amino functionalized group comprising one tertiary amino group but no hydroxyl group.

In a particularly preferred embodiment of the invention, one of the groups R¹ to R³ is a hydroxyl-functionalized group and two of the groups R¹ to R³ are tertiary amino functionalized groups.

Preferably, the tertiary amino functionalized group is a group of formula II 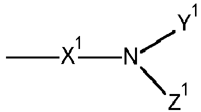 wherein X¹ is a saturated or unsaturated linear or branched aliphatic group comprising from 0 to 3 ether groups and Y¹ and Z¹ independently from one another are a C1- to C4 alkyl group and wherein X¹, Y¹ and Z¹ together have at maximum 20 carbon atoms. In particular, X¹ is a saturated or unsaturated linear or branched aliphatic group of at maximum 10 carbon atoms comprising from 0 or 1 ether group and Y¹ as well as Z¹ each are a methyl group. In a most preferred embodiment X¹ is a saturated linear or branched aliphatic group of at maximum 6 carbon atoms comprising from 0 or 1 ether group and Y¹ as well as Z¹ each are a methyl group.

Preferably, the hydroxyl functionalized group is a group of formula III

-X²-OH

wherein X² is a saturated or unsaturated linear or branched aliphatic group of at maximum 20 carbon atoms comprising from 0 to 3 ether groups. In particular, X² is a saturated or unsaturated linear or branched aliphatic group of at maximum 10 carbon atoms comprising from 0 or 1 ether group. In a most preferred embodiment, X² is a saturated linear or branched aliphatic group of at maximum 6 carbon atoms comprising from 0 or 1 ether group.

As preferred compounds 2-[3,5-bis[3-(dimethylamino)propyl]-1,3,5-triazinan-1-yl]ethanol and 2-[2-[3,5-bis[3-(dimethylamino)propyl]-1,3,5-triazinan-1-yl]ethoxy]ethanol may be mentioned.

The triazinan compounds of formula I may be synthesized by reacting formaldehyde with compounds having a primary amino group, hereinafter referred to as amino compounds.

Instead of formaldehyde as such a compound that releases formaldehyde may be used, for example metaformaldehyde, paraformaldehyde or trioxane.

Preferably, an aqueous solution of formaldehyde is used.

The amino compounds need to have the required structure and substitution to give the compound of formula I. The primary amino group reacts with the formaldyhyde and form the triazinan ring system together with the carbon atoms of the formaldehyde. The remaining part of the amino compounds become groups R¹ to R³ of the triazinan compound of formula I.

Hence the amino compounds correspond to compounds H₂N- R¹, H₂N- R², H₂N- R³ and the 1, 3, 5 - triazinan compound of formula I is obtained by a process comprising reacting formaldehyde and primary amino compounds H₂N- R¹, H₂N- R² and H₂N- R³. All above-metioned provisions regarding R¹ to R³ in formula I apply correspondingly to the amino compounds H₂N- R¹ to H₂N- R³_{.}

The molar relationship of the different amino compounds H₂N- R¹ to H₂N- R³ should correspond to the desired molar relationship of hydroxyl groups to tertiary amino groups in the triazinan of formula I.

According to the stoichiometry of the reaction three moles of amino compounds react with three mols of formaldehyde. The amino compounds and formaldehyde may be used in equimolar amounts; alternatively, either the amino compounds or the formaldehyde may be used in excess. Preferably, the formaldehyde is used in excess.

For example, 0.1 to 10 mols of amino compounds in total may be reacted with 3 mols of formaldehyde; in a preferred embodiment 1 to 5 mols of amino compounds, in a more preferred embodiment 2 to 3 mols of amino compounds are reacted with 3 mols of formaldehyde.

Usually the reaction may be carried out at normal pressure. The reaction is exothermic and the temperature is kept preferably below 50°C, in particular below 40°C. A solvent may be used. In a preferred embodiment the solvent is water in accordance with the preferred use of formaldehyde solution in water. Additional water is formed in the reaction. Water and any other additional solvent used may be distilled off and a product comprising the triazinan compound is obtained.

The obtained product might comprise by-products. In particular, the product may be a mixture of triazane compounds comprising small amounts of triazinane compounds with hydroxyl functionalized groups, only, and/or triazinane compounds with tertiary amino functionalized groups, only. In general, a mixture of trizinan compounds is obtained which comprises at least 70 % by weight of triazinan compounds of formula I, in particular at least 90 %, respectively at least 95 % by weight of triazinan compounds of formula I based on the total weight of all triazinan compounds of the product mixture.

### To the use as catalyst

The triazinan compound of formula I may be used as catalyst in the preparation of polyurethanes or polyureas or mixtures thereof. As triazinan compound the mixture obtained according to the synthesis described above may be used. By-products as triazinan compounds with hydroxyl functionalized groups, only, or triazinan compounds with tertiary amino functionalized groups, only do not have a negative impact on the performance of the triazinan compound of formula I.

Preferred polyureas are polyureas that consist to at least 60 % by weight, in particular of at least 80 % by weight of polyisocyanates and polyamines. Polyisocyanates are defined to be compounds that comprise at least two isocyanate groups. Preferably, they comprise 2 to 6 isocyanate groups. Polyamines are defined to be compounds that comprise at least two amino groups, selected from primary or secondary amino groups. Preferably, they comprise from 2 to 6 amino groups.

Preferred polyurethanes are polyurethanes that consist to at least 60 % by weight, in particular of at least 80 % by weight of polyisocyanates and polyols. Preferred polyisocyanates comprise from 2 to 6 isocyanate groups (see above). Polyols are defined to be compounds that comprise at least two hydroxyl groups. Preferably they comprise from 2 to 6 hydroxyl groups.

In a preferred embodiment the above triazinan compounds are used as catalyst in the preparation of polyurethanes.

Polyurethanes are obtainable by reacting polyisocyanates, polyols and, optionally, further starting materials such as monools, monoamines and polyamines.

Polyureas are obtainable by reacting polyisocyanates, polyamines and, optionally, further starting materials such as mono amines, monools and polyols.

The below definitions, informations and embodiments, including preferred embodiments regarding polyisocyanatea, polyol, polyamines or other compounds relate to polyurethanes as well as to polyureas.

Polyisocyanates may in particular be aliphatic, cycloaliphatic or aromatic polyisocyanates. Mixtures of different polyisocyanates may be used in the preparation of polyurethanes.

Suitable and commonly used polyisocyanates are, for example, 2,2'-, 2,4'- und 4,4'-diphenylmethane diisocyanate (MDI), MDI oligomers or mixtures thereof, isophorone diisocyanate or its oligomers, 2,4- or 2,6-toluene diisocyanate (TDI) or mixtures thereof, tetramethylene diisocyanate or its oligomers, hexamethylene diisocyanate (HDI) or its oligomers, naphtylene diisocyanate or mixtures thereof.

Polyisocyanates may be used in form of polyisocyanate-prepolymers. Polyisocyanate-prepolymers are obtainable by reacting polyisocyanates in molar excess with compounds that have reactive groups such as hydroxy, primary or secondary amino groups.

Suitable polyisocyanates are also dimeric or trimeric adducts of diisocyanates such as uretdiones and isocyanurates. Uretdiones or isocyanurates may be separately synthesized before the preparation of the polyurethane or may be formed during the preparation (in situ). In case of an in situ preparation diisocyanates may be used in excess so that first an uretdione or isocyanurate is formed. The remaining free isocyanate groups of the uretdione or isocyanurate then react with the other starting materials such as polyols or polyamines to give the polyurethane or polyurea.

Suitable polyols are, for example polymeric polyols such as polyetherols or polyesterols.

Suitable polyetherols may, for example, be prepared by reacting epoxides as propylene oxide or ethylene oxide or tetrahydrofurane with a starting material having an active hydrogen atom, as, for example, a compound with a hydroxy group, a primary or secondary amino group or a carboxylic acid group or water.

Suitable polyesterols may, for example, be prepared by reacting aliphatic or aromatic dicarboxylic acids with diols or polyols.

In a preferred embodiment of the invention polyetherols are used as polymeric polyols.

In addition to polymeric polyols low molecular weight polyols may be used as chain extenders or crosslinkers. Low molecular weight polyols may be selected, for example from alkane diols or alkane triols.

Other starting materials are compounds with only one reactive group, such as mono-ols or mono-amines. Such compounds form non reactive end groups of the polymeric chain and limit the molecular weight.

Triazinan compounds of formula I may be used as catalyst in the preparation of the fore standing polyurethanes or polyureas. The triazinan compounds may be admixed to the starting materials and reaction of the starting materials occurs in presence of the triazinan compounds.

Other catalysts may be used in addition to the triazinan compounds of formula I. Such other catalysts may be other amino compounds or organo-metallic compounds.

In a preferred embodiment of the invention at least 30 % by weight, in particular at least 50 % by weight, more preferred at least 80 % by weight of all catalysts used are triazinan compounds of formula I.

In a particularly preferred embodiment of the invention no other catalysts are used and triazinan compounds of formula I are used, only.

Preferably, the catalysts are used in an amount of 0.001 bis 20 parts by weight, in particular of 0.05 bis 10 parts by weight on 100 parts by weight of polyisocyanats. This applies both, to the preparation of polyurethanes as well as to the preparation of polyurea.

For the preparation of polyurethanes or polyureas two components may be formed. One component comprising all compounds with isocyanate groups, in particular the polyisocyanates, and optionally additives, and a further component comprising all compounds that are reactive with isocyanate, in particular the compounds with hydroxyl groups and primary or secondary amino groups, and further additives. The catalysts may be added to both components or to one of the components, only. After mixing of the components reaction occurs, which may usually be at room temperature.

In the case of polyurethane foams a blowing agent is usually used as additive. The blowing agent may be added to both components or to one of the components, only.

Blowing agents may be chemical compounds that react with isocyanate and form gaseous reaction products. These are so called chemical blowing agents. Chemical blowing agents are for example water and carboxylic acids. Blowing agents may also be compounds that are solved or emulsified in the above components and simply evaporate under the reaction conditions, so called physical blowing agents. Physical blowing agents are, for example hydrocarbons as pentane or fluorinated hydrocarbons.

Further additives that may be added to the above components are, for example, flame retardants, stabilizers, biocides, pigments, colorants, fillers, release agents, etc.

The polyurethane formed may be a thermoplastic polyurethane, a duromer, a polyurethane foam, which includes a soft polyurethane foam, a semi-rigid polyurethane foam or a rigid polyurethane foam.

In a preferred embodiment of the invention the obtained polyurethane is a polyurethane foam. Polyurethane foams have an average density of from 20 to 850 grams per liter.

The trazinan compounds of formula I can be easily and effectively prepared. They have high suitability as catalysts for the preparation of polyurethanes and polyureas. Furthermore they are bonded via the hydroxyl group of at least one of the groups R¹ to R³ to the obtained polyurethane or polyurea and do not leak by time from the polymer or the end product of the final application which may, for example, be a mold or seal.

### Example 1

### Synthesis of 2-[3,5-bis[3-(dimethylamino)propyl]-1,3,5-triazinan-1-yl]ethanol.

689.4 g (6.73 Mol) of N,N-Dimethylaminopropylamine and 206.0 g (3.37 Mol) of Ethanolamine were put in a 2.5 liter mini reactor and stirred for 10 minutes. The temperature of the reactor was kept constant at 35°C. To the mixture of N,N-Dimethylaminopropylamine and Ethanolamine 1001.0 g (10.00 Mol) of 30% formaldehyde solution in water were added within five hours under permanent stirring. The mixture was stirred for next four hours at 40°C. On the next day 783 g of water were distilled off at 125 mbar. Subsequently, in order to remove traces of low boiling components the temperature was set to 75°C and the distillation pressure was reduced down to 10 mbar. As a result 997.0 g of a clear, colorless, slightly viscous liquid were obtained.
Content: 85.9% (NMR)
Yield: 85.0% structure of 2-[3,5-bis[3-(dimethylamino)propyl]-1,3,5-triazinan-1-yl]ethanol:

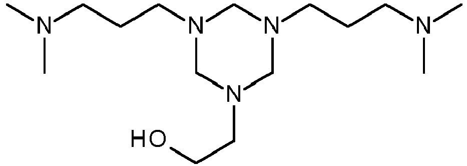

### Example 2

### Synthesis of 2-[2-[3,5-bis[3-(dimethylamino)propyl]-1,3,5-triazinan-1-yl]ethoxy]ethanol.

688.3g (6.73 Mol) of N,N-Dimethylaminopropylamine and 357.9 g (3.37 Mol) of 2-(2-Aminoethoxy)ethanol were put in a 2.5 liter mini reactor and stirred for 10 minutes. The temperature of the reactor was kept constant at 30°C. To the mixture of N,N-Dimethylaminopropylamine and 2-(2-Aminoethoxy)ethanol 1001.0 g (10.00 Mol) of 30% formaldehyde solution in water were added within five hours under permanent stirring. The mixture was then stirred for twelve hours at 30°C. On the next day 865 g of water were distilled off at 125 mbar. Subsequently, in order to remove traces of low boiling components the temperature was set to 75°C and the distillation pressure was reduced down to 10 mbar. As a result 1155.0 g of a clear, colorless, slightly viscous liquid were obtained.
Content: 96.8% (NMR)
Yield: 97.0%

Structure of 2-[2-[3,5-bis[3-(dimethylamino)propyl]-1,3,5-triazinan-1-yl]ethoxy]ethanol:

## Claims

**1.** A 1, 3, 5 - triazinan compound of formula I 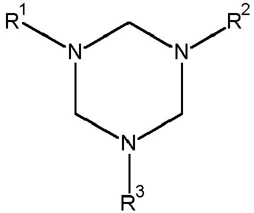 wherein the groups R¹ to R³ are organic groups and wherein at least one of the groups R¹ to R³ is a hydroxyl functionalized group comprising at least one hydroxyl group and wherein at least one of the groups R¹ to R³ is a tertiary amino functionalized group comprising at least one tertiary amino group.

**2.** A triazinan compound according to claim 1, wherein the groups R¹ to R³ are non aromatic hydrocarbon groups of at maximum 20 carbon atoms which may comprise ether groups and wherein at least one of the groups R¹ to R³ is a hydroxyl functionalized group comprising one hydroxyl group but no tertiary amino group and at least one of the groups R¹ to R³ is a tertiary amino functionalized group comprising one tertiary amino group but no hydroxyl group.

**3.** A triazinan compound according to any of claims 1 or 2, wherein at least one of the groups R¹ to R³ is a tertiary amino functionalized group of formula II 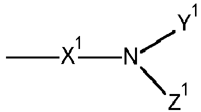 wherein X¹ is a saturated or unsaturated linear or branched aliphatic group comprising from 0 to 3 ether groups and Y¹ and Z¹ independently from one another are a C1- to C4 alkyl group and wherein X¹, Y¹ and Z¹ together have at maximum 20 carbon atoms

**4.** A triazinan compound according to claim 1 to 3, wherein at least one of the groups R¹ to R³ is a hydroxyl functionalized group of formula III
-X²-OH
wherein X² is a saturated or unsaturated linear or branched aliphatic group of at maximum 20 carbon atoms comprising from 0 to 3 ether groups

**5.** A triazinan compound according to any of claims 1 to 4, wherein one of the groups R¹ to R³ is a hydroxyl-functionalized group and two of the groups R¹ to R³ are tertiary amino functionalized groups

**6.** A triazinan compound according to any of claims 1 to 4, wherein the triazinan compound is 2-[3,5-bis[3-(dimethylamino)propyl]-1,3,5-triazinan-1-yl]ethanol or 2-[2-[3,5-bis[3-(dimethylamino)propyl]-1,3,5-triazinan-1-yl]ethoxy]ethanol.

**7.** Mixture of triazinan compounds comprising at least 70 % by weight of triazinan compounds of formula I based on the total weight of all triazinan compounds

**8.** Process for the preparation of a 1, 3, 5 - triazinan compound of formula I comprising reacting formaldehyde and primary amino compounds R¹-NH₂, R²-NH₂ and R³-NH₂ wherein R¹ to R³ have the meaning as in formula I

**10.** Use of a 1, 3, 5 - triazinan compound of formula I as catalyst for the preparation of polyurethanes or polyureas
